# EUROPÄISCHE PATENTSCHRIFT

(11) **EP 1 886 703 B1**
(45) Veröffentlichungstag und Bekanntmachung des Hinweises auf die Patenterteilung: **23.06.2010**
(21) Anmeldenummer: 07013429.1
(22) Anmeldetag: 10.07.2007
(51) Int. Cl.: A61L 31/02, A61L 31/14, A61L 31/16, A61L 31/10

(54) **Stent mit einer genisteinhaltigen Beschichtung oder Kavitätenfüllung**
Stent with a coating containing genistein or a cavity filling
Stent doté d'un revêtement contenant de la geneste ou d'une enveloppe de cavité

(30) Priorität: 07.08.2006 DE 102006038241
(43) Veröffentlichungstag der Anmeldung: 13.02.2008
(73) Patentinhaber: Biotronik VI Patent AG, 6341 Baar (CH)
(72) Erfinder: Sternberg, Katrin, Dr., 18057 Rostock (DE); Schmitz, Klaus-Peter, Prof. Dr., 18119 Warnemünde (DE); Behrend, Detlef, Prof. Dr., 18119 Rostock (DE); Hennighausen, Gerhard, Prof. Dr., 18069 Lambrechtshagen (DE); Martini, Claus, Dr., 8044 Zürich (CH); Harder, Claus, Dr., 91080 Uttenreuth (DE)
(74) Vertreter: Lindner-Vogt, Karin L.

(56) Entgegenhaltungen:
- WO-A-2006/060033
- DE-A1- 10 253 634
- JP-A- 9 056 807

## Beschreibung

Die Erfindung betrifft (i) einen Stent aus einem biokorrodierbaren metallischen Werkstoff, der eine Beschichtung oder Kavitätenfüllung aufweist, die aus einem Wirkstoff besteht oder diesen enthält, sowie (ii) eine neue Verwendung von Genistein.

Die Implantation von Stents hat sich als eine der wirkungsvollsten therapeutischen Maßnahmen bei der Behandlung von Gefäßerkrankungen etabliert. Stents haben den Zweck, in Hohlorganen eines Patienten eine Stützfunktion zu übernehmen. Stents herkömmlicher Bauart weisen dazu eine filigrane Tragstruktur aus metallischen Streben auf, die zur Einbringung in den Körper zunächst in einer komprimierten Form vorliegt und am Ort der Applikation aufgeweitet wird. Einer der Hauptanwendungsbereiche solcher Stents ist das dauerhafte oder temporäre Weiten und Offenhalten von Gefäßverengungen, insbesondere von Verengungen (Stenosen) der Herzkranzgefäße. Daneben sind beispielsweise auch Aneurysmenstents bekannt, die zur Stützung beschädigter Gefäßwände dienen.

Stents besitzen eine Umfangswandung von ausreichender Tragkraft, um das verengte Gefäß im gewünschten Maße offen zu halten, und einen rohrförmigen Grundkörper durch den der Blutfluss ungehindert weiterläuft. Die tragende Umfangswandung wird in der Regel von einer gitterartigen Tragstruktur gebildet, die es erlaubt, den Stent in einem komprimierten Zustand mit kleinem Außendurchmesser bis zur behandelnden Engstelle des jeweiligen Gefäßes einzuführen und dort beispielsweise mit Hilfe eines Ballonkatheters soweit aufzuweiten, dass das Gefäß den gewünschten, vergrößerten Innendurchmesser aufweist. Um unnötige Gefäßbeschädigungen zu vermeiden, sollte der Stent nach dem Aufweiten und nach Entfernen des Ballons nicht oder nur in geringem Umfang elastisch zurückfedern, so dass der Stent beim Aufweiten nur wenig über den gewünschten Enddurchmesser hinaus geweitet werden muss. Weitere Kriterien, die in Bezug auf einen Stent wünschenswert sind, umfassen beispielsweise eine gleichmäßige Flächenabdeckung und eine Struktur, die eine gewisse Flexibilität in Bezug auf die Längsachse des Stents erlaubt. In der Praxis wird zur Realisation der genannten mechanischen Eigenschaften der Stent in der Regel aus einem metallischen Werkstoff geformt.

Neben den mechanischen Eigenschaften eines Stents sollte dieser aus einem biokompatiblen Material bestehen, um Abstoßungsreaktionen vorzubeugen. Derzeit werden bei etwa 70 % aller perkutanen Interventionen Stents eingesetzt, in 25% aller Fälle kommt es jedoch zu einer In-Stent Restenose aufgrund eines überschießenden neointimalen Wachstums, das durch eine starke Proliferation der arteriellen glatten Muskelzellen und eine chronische Entzündungsreaktion hervorgerufen wird. Zur Senkung der Restenoseraten werden verschiedene Lösungsansätze verfolgt.

Ein Ansatz zur Minderung der Restenoserate sieht vor, auf dem Stent eine pharmazeutisch aktive Substanz (Wirkstoff) bereitzustellen, die den Mechanismen der Restenose entgegenwirkt und den Heilungsverlauf unterstützt. Der Wirkstoff wird in Reinform oder eingebettet in eine Trägermatrix als Beschichtung aufgetragen oder in Kavitäten des Implantats gefüllt. Beispiele umfassen die Wirkstoffe Sirolimus und Paclitaxel.

Ein weiterer aussichtsreicher Ansatz zur Lösung des Problems liegt in der Verwendung biokorrodierbarer Metalle und deren Legierungen, denn zumeist ist eine dauerhafte Stützfunktion durch den Stent nicht erforderlich; das zunächst geschädigte Körpergewebe regeneriert. So wird beispielsweise in DE 197 31 021 A1 vorgeschlagen, medizinische Implantate aus einem metallischen Werkstoff zu formen, dessen Hauptbestandteil Eisen, Zink oder Aluminium sind bzw. ein Element aus der Gruppe der Alkalimetalle oder Erdalkalimetalle. Als besonders geeignet werden Legierungen auf Basis von Magnesium, Eisen und Zink beschrieben. Nebenbestandteile der Legierungen können Mangan, Kobalt, Nickel, Chrom, Kupfer, Cadmium, Blei, Zinn, Thorium, Zirkonium, Silber, Gold, Palladium, Platin, Silizium, Calcium, Lithium, Aluminium, Zink und Eisen sein. Weiterhin ist aus der DE 102 53 634 A1 der Einsatz einer biokorrodierbaren Magnesiumlegierung mit einem Anteil von Magnesium >90%, Yttrium 3,7 - 5,5%, Seltenerdmetallen 1,5 - 4,4% und Rest < 1 % bekannt, die sich insbesondere zur Herstellung einer Endoprothese, z. B. in Form eines selbstexpandierenden oder ballonexpandierbaren Stents, eignet.

Der Einsatz von biokorrodierbaren metallischen Werkstoffen, insbesondere biokorrodierbaren Magnesium- oder Eisenlegierungen in Stents dürfte zu einer deutlichen Minderung der Restenoserate führen. Ungeachtet der erreichten Fortschritte sind jedoch noch Teilprobleme zu lösen: So kann der durch die Korrosion des Werkstoffs bedingte Anstieg des pH-Werts und dadurch induzierte Calciumstrom in die umgebenden Zellen zu einer unerwünschten Kontraktion der Gefäßwand (Gefäßspasmen) führen.

Der Erfindung liegt die Aufgabe zugrunde, die geschilderten Nachteile des Standes der Technik zu überwinden. Insbesondere soll möglicherweise den durch den Anstieg des pH-Werts induzierten Gefäßspasmen vorgebeugt werden.

Ein erster Aspekt der Erfindung liegt in der Bereitstellung eines Implantats aus einem biokorrodierbaren metallischen Werkstoff mit einer Beschichtung oder Kavitätenfüllung, die aus Genistein besteht oder dieses enthält.

Genistein (5,7-Dihydroxy-3-(4-hydroxyphenyl)-4*H*-1-benzopyran-4-on, 4',5,7-Trihydroxyisoflavon, auch Differenol A, Prunetol, Sophoricol genannt) ist ein Phytoöstrogen aus der Gruppe der Isoflavone und ein Sekundärmetabolit aus Pflanzen (Leguminosen, Papilionoiden, Rosaceen etc.; häufig in glycosylierter Form), wurde jedoch auch in Kulturen von Mikroorganismen (Actinomyceten, Aspergillus, Mycobacterien u.a.) gefunden. Genistein weist die nachfolgende Strukturformel auf:

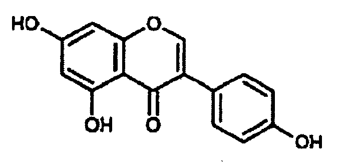

Genistein wird über die Nahrung aufgenommen und lässt sich im Serum von Menschen nachweisen. Folgende pharmakologische Wirkungen und Wirkungsmechanismen werden dem Genistein zugeschrieben:
- ein Agonist am Estrogen-β-Rezeptor;
- Hemmstoff von Tyrosinkinasen (u.a. von EGF-Rezeptor-Tyrosinkinase);
- Hemmstoff von Topoisomerasen (insbesondere Topoisomerase II);
- Hemmung kardialer L-Typ Ca²⁺-Kanäle;
- Hemmung von NFkappa B
bei Endothelzellen:
- Hemmung der Expression des Vascular Endothelial Growth Factor (VEGF);
- Hemmung der Collagen-induzierten Plättchenaggregation;
- Hemmung der Sekretion von Protein-1;
- Hemmung der Integrin-abhängigen Leukozytenadhäsion an Endothelzellen;
- Hemmung der Adhäsion von Monozyten an Endothelzellen nach TNFα-Stimulation in Abhängigkeit von Blutfluss/physikalischen Kräften;
- Hemmung der in Endothelzellen durch Aldosteron ausgelösten Expression von ACE;
- Hemmung der durch Homocystein induzierten Veränderungen im Proteom von humanen Endothelzellen, die in Metabolismus, Entgiftung und Genregulation Funktionen haben;

Wirkungen auf vaskuläre glatte Muskelzellen:
- Hemmung der Proliferation (insbesondere über Hemmung von Tyrosinkinasen);
- Hemmung der durch oxidativen Stress induzierten Migration;
- Hemmung der Endothelin-1-Wirkung auf den Ca²⁺-Influx in glatten Muskelzellen von Coronarien;

Ferner sind pharmakologische Effekte von Genistein auf Tumorzellen und pathophysiologische Vorgänge in der Postmenopause beschrieben.

Es hat sich nun überraschenderweise gezeigt, dass Genistein ein höheres Potential zur Wachstumsinhibierung von humanen glatten Muskelzellen und zur Wachstumsförderung von Endothelzellen besitzt als das humane Östrogen 17β-Estradiol. Weiterhin hat Genistein - neben seiner auf humane glatte Muskelzellen / humane Endothelzellen spezifischen biologischen Wirkung - als Hemmstoff der Proteine Tyrosinkinase und Topoisomerase II den Vorteil, dass kardiale L-Typ Ca²⁺-Kanäle gehemmt werden. Änderungen der intrazellulären Calciumkonzentration, die durch die Calcium-Kanäle mehr oder weniger selektiv gesteuert werden, sind entscheidend für viele physiologische Prozesse. Sie führen unter anderem zur Synthese und Sekretion von Hormonen, regulieren die Expression von Genen und steuern Enzymaktivitäten. So genannte Calciumkanalblocker, hier in Form von Genistein, regulieren die Kontraktionskraft der Herzmuskulatur und der glatten Muskulatur der Gefäße (in der Regel mindern sie diese), indem sie den Calciumeinstrom in die Muskelzellen hemmen. Diese Hemmung ist für biokorrodierbare Stents auf der Basis der genannten Metalllegierungen (s. Kapitel Hintergrund der Erfindung und Stand der Technik) besonders vorteilhaft, denn so kann einer unerwünschten Kontraktion der Gefäßwand (Gefäßspasmen) vorgebeugt werden. Schließlich deuten erste Untersuchungen darauf hin, dass die Gegenwart von Genistein die Umsetzung von zunächst bei der Korrosion anfallenden Metallhydroxiden, insbesondere Magnesium- oder Eisenhydroxid, zu entsprechenden Phosphaten unterstützt.

Genistein kann im Zusammenhang mit biokorrodierbaren metallischen Werkstoffen, insbesondere Magnesium- oder Eisenlegierungen, deshalb eingesetzt werden, weil der mit der Korrosion des Wirkstoffs verbundene pH-Wert-Anstieg keinen Einfluss auf die chemische Stabilität des Moleküls hat. Hinweise auf eine durch den pH-Wert-Anstieg induzierte Metabolisierung des Wirkstoffs Genistein wurde nach Untersuchungen der Anmelderin nicht gefunden.

Eine Beschichtung im Sinne der Erfindung ist eine zumindest abschnittsweise Auftragung der Komponenten auf den Grundkörper des Stents. Vorzugsweise wird die gesamte Oberfläche des Grundkörpers des Stents von der Beschichtung bedeckt. Alternativ kann das Genistein in einer Kavität des Stents bereitgestellt werden.

Vorzugsweise ist das Genistein in eine biodegradierbare organische Trägermatrix eingebettet ist. Besonders bevorzugt ist die biodegradierbare organische Trägermatrix ein Polymer ausgewählt aus der Gruppe Polyglycolide, Polylactide, Polyhydroxybuttersäure und Poly-ε-caprolacton. Insbesondere ist die biodegradierbare organische Trägermatrix Poly-L-lactid.

Genistein eignet sich als lipophiler Wirkstoff besonders zur Verarbeitung in biodegradierbaren organischen Trägermatrizes mit hydrophobem Charakter. Der Wirkstoff ist aufgrund seines lipophilen Charakters in organischen Lösungsmitteln gut löslich. Dies erleichtert die Wirkstoffinkorporation in die polymere Trägermatrix und unterstützt eine homogene und reproduzierbare Wirkstoffverteilung in dieser Trägermatrix.

Die Beschichtung / Füllung kann folgende Zusatzstoffe enthalten:
- Lipophile Vitamine (Vitamin A, D, E, K)
- Fettsäuren (Linol-, Öl-, Palmitin-, Stearin-, Benzoe-, Zimt-, Linolen-, Arachidon-, Myristin-, Arachin-, Behen-, Palmitolein-, Elaidin-, Vaccen-, Icosen-, Cetolein-, Eruca-, Nervonsäure)

- Antioxidantien (Alpha-Tocopherol E 307, Ascorbinsäure E 300, Ascorbylpalmitat E 304, Butylhydroxytoluol (BHT) E 321, Butylhydroxyanisol (BHA), Calcium-Dinatrium-EDTA E 385, Calcium-L-Ascorbat E 302, Calciumhydrogensulfit E 227, Calciumsulfit E 226, Citronensäure E 330, Delta-Tocopherol E 309, Diphosphate E 450, Dodecylgallat, Laurylgallat E 312, Gamma-Tocopherol E 308, Isoascorbinsäure E 315, Kaliumbisulfit E 228, Kaliumcitrat E 332, Kaliumsulfit E 224, Lecithin E 322, Milchsäure E 270, Natrium-L-Ascorbat E 301, Natrium-L-Ascorbat E 301, Natriumbisulfit E 222, Natriumcitrat E 331, Natriumdisulfit E 223, Natriumisoascorbat E 316, Natriumsulfit E 221, Octylgallat E 311, Polyphosphate E 452, Propylgallat E 310, Schwefeldioxid E 220, Tocopherol E 306, Triphosphate E 451, Zinn-II-Chlorid E 512)
- Emulgatoren (Ammoniumphoshatide E 442, Ascorbylpalmitat E 304, Calciumphosphat E 341, Calciumstearoyl-2-lactylat E 482, Citronensäureester von Mono- und Diglyceriden von Speisefettsäuren E 472c, Diphosphate E 450, Kaliumphosphat E 340, Lecithin E 322, Natriumphosphat E 339, Natriumstearoyl-2-lactylat E 481, Phosphorsäure E 338, Polyglycerin-Polyricinoleat E 476, Polyoxyethylen (40) stearat E 431, Polyphosphate E 452, Polysorbat 20 E 432, Polysorbat 40 E 434, Polysorbat 60 E 435, Polysorbat 65 E 436, Polysorbat 80 E 433, Propylenglycolalginat E 405, Sorbitanmonolaurat E 493, Sorbitanmonooleat E 494, Sorbitanmonopalmitat E 495, Sorbitanmonostearat E 491, Sorbitantristearat E 492, Stearyltartrat E 483, Triphosphate E 451, Zuckerglyceride E 474)
- Phospholipide
- Fluoreszenzmarker
- Röntgenmarker
- Farbstoffe

Als biokorrodierbar im Sinne der Erfindung werden metallische Werkstoffe bezeichnet, bei denen in physiologischer Umgebung ein Abbau stattfindet, der letztendlich dazu führt, dass das gesamte Implantat oder der aus dem Werkstoff gebildete Teil des Implantates seine mechanische Integrität verliert. Biokorrodierbare metallische Werkstoffe im Sinne der Erfindung umfassen insbesondere Metalle und Legierungen ausgewählt aus der Gruppe der Elemente Eisen, Wolfram und Magnesium.

Vorzugsweise ist der biokorrodierbare Werkstoff eine Magnesium- oder Eisenlegierung. Besonders bevorzugt ist eine biokorrodierbare Magnesiumlegierung, welche Yttrium und weitere Seltenerdmetalle enthält, da sich eine derartige Legierung aufgrund ihrer physikochemischen Eigenschaften und hohen Biokompatibilität, insbesondere auch seiner Abbauprodukte, auszeichnet.

Besonders bevorzugt wird eine Magnesiumlegierung der Zusammensetzung Seltenerdmetalle 5,2 - 9,9 Gew.%, davon Yttrium 3,7 - 5,5 Gew.%, und Rest < 1 Gew.%, wobei Magnesium den auf 100 Gew.% fehlenden Anteil an der Legierung einnimmt, eingesetzt. Diese Magnesiumlegierung bestätigte bereits experimentell und in ersten klinischen Versuchen ihre besondere Eignung, d. h. zeigt eine hohe Biokompatibilität, günstige Verarbeitungseigenschaften, gute mechanische Kennwerte und ein für die Einsatzzwecke adäquates Korrosionsverhalten. Unter der Sammelbezeichnung "Seltenerdmetalle" werden vorliegend Scandium (21), Yttrium (39), Lanthan (57) und die 14 auf Lanthan (57) folgenden Elemente, nämlich Cer (58), Praseodym (59), Neodym (60), Promethium (61), Samarium (62), Europium (63), Gadolinium (64), Terbium (65), Dysprosium (66), Holmium (67), Erbium (68), Thulium (69), Ytterbium (70) und Lutetium (71), verstanden.

Die metallischen Werkstoffe bzw. Magnesium- oder Eisenlegierungen sind so in ihrer Zusammensetzung zu wählen, dass sie biokorrodierbar sind. Als Prüfmedium zur Testung des Korrosionsverhaltens einer in Frage kommenden Legierung dient künstliches Plasma, wie es nach EN ISO 10993-15:2000 für Biokorrosionsuntersuchungen vorgeschrieben ist (Zusammensetzung NaCl 6,8 g/l, CaCl2 0,2 g/l, KCI 0,4 g/l, MgSO₄ 0,1 g/l, NaHCO₃ 2,2 g/l, Na₂HPO₄ 0,126 g/l, NaH₂PO₄ 0,026 g/l). Eine Probe der zu untersuchenden Legierung wird dazu in einem verschlossenen Probenbehälter mit einer definierten Menge des Prüfmediums bei 37°C gelagert. In zeitlichen Abständen - abgestimmt auf das zu erwartende Korrosionsverhalten - von wenigen Stunden bis zu mehreren Monaten werden die Proben entnommen und in bekannter Weise auf Korrosionsspuren untersucht. Das künstliche Plasma nach EN ISO 10993-15:2000 entspricht einem blutähnlichen Medium und stellt damit eine Möglichkeit dar, eine physiologische Umgebung im Sinne der Erfindung reproduzierbar nachzustellen.

Ein zweiter Aspekt der Erfindung bezieht sich auf die Verwendung von Genistein als Beschichtungsmaterial für einen Stent aus einem biokorrodierbaren Werkstoff, insbesondere einer biokorrodierbaren Magnesium- oder Eisenlegierung.

Stents aus der biokorrodierbaren Magnesiumlegierung WE43 (97 Gew.% Magnesium, 4 Gew.% Yttrium, 3 Gew.% Seltenerdmetalle außer Yttrium) wurden wie folgt beschichtet:

Die Magnesiumoberflächen der Stents wurden durch Behandlung mit einem Argonplasma aufgeraut, um eine höhere Haftigkeit des Wirkstoffs auf der Stentoberfläche zu erzielen.

Es wurde eine 5×10⁻² M methanolische Lösung von Genistein angesetzt und durch Sprühen direkt aufgetragen. Die Stents wurden manuell auf einen Ballon gekrimpt und bei Raumtemperatur getrocknet. Es wurden pro Stent ca. 120 mg Genistein aufgetragen.

Die Stents wurden zur Freisetzung in porcinem Plasma jeweils in ein schraubdeckelverschließbares Glasvial überführt, mit 1 ml porcinem Plasma versetzt, verschlossen und bei 37°C in einem Brutschrank maschinell geschüttelt. Zu vorgegebenen Probeentnahmezeitpunkten wurden die Stents jeweils entnommen und in einem neuen Vial mit dem entsprechenden Volumen des frischen Elutionsmittels dem Elutionsvorgang weiterhin unterzogen. Von der verbliebenen Plasmaprobe wurden 0,5 ml in einem schraubdeckelverschließbaren Glasvial mit 3 ml Diethylether versetzt, 5 min geschüttelt, die Plasmaphase im Gefrierschrank ausgefroren, der Diethylether abgetrennt und zur Trockne eingedampft sowie der Rückstand mit 0,5 ml Laufmittel (0,1 Gew.% wässrige Orthophosphorsäure, Acetonitril (62/38)) aufgenommen. Die jeweils resultierende Lösung wurde mittels HPLC vermessen.

Die HPLC-Untersuchungen zeigten, dass die Freisetzung des Wirkstoffs in porcinem Plasma bereits nach ca. 10 min abgeschlossen ist. Hinweise auf eine Instabilität des Wirkstoffs Genistein im Zusammenhang mit dem Kontakt des Genisteins auf der Magnesiumoberfläche sind weder in den Massebilanzen des Wirkstoffs noch in den Chromatogrammen zu erkennen gewesen.

Weitere Untersuchungen zur Freisetzungskinetik von Genistein erfolgten aus einer polymeren Trägermatrix, genauer Poly-L-lactid. Genistein wurde mit einem Gewichtsanteil von 30, 50 und 60 Gew.% bezogen auf das Gesamtgewicht von Genistein und Poly-L-lactid eingesetzt. Die Untersuchungen wurden in porcinem Blutplasma durchgeführt und in analoger Weise zu den bereits obig genannten Arbeitsschritten ohne Polymermatrix durchgeführt.

Es zeigte sich, dass zum einen Poly-L-lactid eine für die Freisetzung von Genistein geeignete biodegradierbare Trägermatrix darstellt und zum anderen eine deutliche Verzögerung der Wirkstofffreisetzung gegenüber dem Auftragen des reinen Wirkstoffs erzielt werden kann. So wurde die Hauptmenge des inkorporierten Genisteins aus der Poly-L-lactid-Trägermatrix nach etwa 3,5 Monaten eluiert.

## Patentansprüche

1. Stent aus einem biokorrodierbaren metallischen Werkstoff und mit einer Beschichtung oder Kavitätenfüllung, die aus Genistein besteht oder dieses enthält.

2. Stent nach Anspruch 1, bei dem der biokorrodierbare metallische Werkstoff eine Magnesium- oder Eisenlegierung ist.

3. Stent nach Anspruch 1 oder 2, bei dem das Genistein in eine biodegradierbare organische Trägermatrix eingebettet ist.

4. Stent nach Anspruch 3, bei dem die biodegradierbare organische Trägermatrix ein Polymer ausgewählt aus der Gruppe Polyglycolide, Polylactide, Polyhydroxybuttersäure und Poly-ε-caprolacton ist.

5. Stent nach Anspruch 4, bei dem die biodegradierbare organische Trägermatrix Poly-L-lactid ist.

6. Verwendung von Genistein als Beschichtungsmaterial für einen Stent aus einem biokorrodierbaren metallischen Werkstoff.

## Claims

1. A stent of a biocorrodible metallic material and a coating or cavity filling, comprising or containing genistein.

2. The stent according to claim 1, wherein the biocorrodible metallic material is a magnesium or iron alloy.

3. The stent according to claim 1 or 2, wherein the genistein is embedded in a biodegradable organic carrier matrix.

4. The stent according to claim 3, wherein the biodegradable organic carrier matrix is a polymer selected from the group of polyglycolides, polylactides, polyhydroxybutyric acid and poly-ε-caprolactone.

5. The stent according to claim 4, wherein the biodegradable organic carrier matrix is poly-L-lactide.

6. The use of genistein as a coating material for a stent of a biocorrodible metallic material.

## Revendications

1. Stent en matériau métallique biocorrodable et comportant un revêtement ou remplissage de cavité composé de génistéine ou en contenant.

2. Stent selon la revendication 1, dans lequel le matériau métallique biocorrodable est un alliage de magnésium ou de fer.

3. Stent selon la revendication 1 ou 2, dans lequel la génistéine est noyée dans une matrice porteuse organique biodégradable.

4. Stent selon la revendication 3, dans lequel la matrice porteuse organique biodégradable est un polymère sélectionné dans le groupe des polyglycolides, des polylactides, des acides polyhydroxybutyriques et du poly-ε-caprolactone.

5. Stent selon la revendication 4, dans lequel la matrice porteuse organique biodégradable est un poly-L-lactide.

6. Utilisation de génistéine comme matériau de revêtement pour un stent en matériau métallique biocorrodable.
